# EUROPÄISCHE PATENTSCHRIFT

(11) **EP 2 775 941 B1**
(45) Veröffentlichungstag und Bekanntmachung des Hinweises auf die Patenterteilung: **09.01.2019**
(21) Anmeldenummer: 12786922.0
(22) Anmeldetag: 06.11.2012
(51) Int. Cl.: A61B 17/11

(54) **ANASTOMOSEGERÄT MIT KOLLABIERBAREM DISTALEM KOPFELEMENT**
ANASTOMOSIS DEVICE WITH COLLAPSIBLE DISTAL HEAD ELEMENT
APPAREIL D'ANASTOMOSE POURVU D'UN D'ÉLÉMENT DE TÊTE DISTAL POUVANT ÊTRE REPLIÉ

(30) Priorität: 10.11.2011 DE 102011055236
(43) Veröffentlichungstag der Anmeldung: 17.09.2014
(73) Patentinhaber: Aesculap AG, 78532 Tuttlingen (DE)
(72) Erfinder: WEISSHAUPT, Dieter, 78194 Immendingen (DE); ROTHWEILER, Christoph, 78166 Donaueschingen (DE)
(74) Vertreter: Winter, Brandl, Fürniss, Hübner, Röss, Kaiser, Polte - Partnerschaft mbB
(86) Internationale Anmeldenummer: PCT/EP2012/071941
(87) Internationale Veröffentlichungsnummer: WO 2013/068354

(56) Entgegenhaltungen:
- DE-U1-202010 013 152
- US-A- 4 700 703
- US-A- 4 893 622
- US-A1- 2002 025 243
- US-A1- 2006 111 704

## Beschreibung

Die vorliegende Erfindung betrifft ein chirurgisches Instrument zum Verbinden von Körpergewebe mit einem Schaft und einem ersten und einem zweiten Werkzeugelement, welche Werkzeugelemente relativ zueinander bewegbar angeordnet oder ausgebildet sind und jeweils eine Elektrode umfassen, welche Elektroden in einer Annäherungsstellung der Werkzeugelemente einen minimalen Abstand voneinander definieren, einander gegenüberliegen und aufeinander zu weisen, wobei das erste Werkzeugelement am distalen Ende des Schafts angeordnet oder ausgebildet ist.

Chirurgische Instrumente der eingangs beschriebenen Art sind beispielsweise aus DE 20 2010 013 152 U1 bekannt. Mit ihnen lassen sich Hohlorgane, beispielsweise Gefäße oder Darmabschnitte, schonend und ohne Verwendung von Klammern oder Nahtmaterial miteinander verbinden. Insbesondere sind End- zu-End-, Seit-zu-End und Seit-zu-Seit- Anastomosen durchführbar.

Weiterhin ist ein chirurgisches Instrument zum Herstellen arterio-venöser Fisteln aus der Druckschrift US2006/0111704 A1 bekannt.

Zudem offenbart die Druckschrift DE102009059196A1 ein chirurgisches System zum Verbinden von Körpergewebe, umfassend ein chirurgisches Instrument mit zwei relativ zueinander bewegbaren Werkzeugelementen, welche jeweils eine HF-Elektrode umfassen, die in einer Annäherungsstellung der Werkzeugelemente einen minimalen Abstand voneinander definieren, einander gegenüberliegen und aufeinander zu weisen, wobei eine Überdehnung insbesondere mittels eines Stromflusses erzeugter Körpergewebeteilverbindungen beim Entfernen des chirurgischen Instruments vermieden wird, indem das Instrument einen Schaft aufweist, an dessen distalem Ende mindestens ein erstes der Werkzeugelemente angeordnet oder ausgebildet ist, und ein zweites Werkzeugelement von einer Betriebsstellung, in welcher es in die Annäherungsstellung bringbar ist, in eine Entfernungsstellung und/oder umgekehrt bringbar ist, in welcher Entfernungsstellung ein Flächenbereich einer senkrechten Projektion des zweiten Werkzeugelementes auf eine Projektionsebene, welche senkrecht zur Schaftrichtung im Bereich des zweiten Werkzeugelements verläuft, kleiner ist als in der Betriebsstellung.
Aus der Druckschrift US4700703 ist weiterhin ein chirurgisches Tackerinstrument mit einer Tackerpatronenanordnung bekannt.
Weiterhin offenbart auch die Druckschrift US4893622 ein Instrument zum Tackern von Hohlorganen.

Ebenso offenbart die Druckschrift US2002/0025243 A1 ein chirurgisches Tackerinstrument, um eine Hohlstruktur an eine andere Struktur zu tackern.

Ein Problem bei derartigen Instrumenten ist es, diese, insbesondere deren zweite Werkzeugelemente, nach dem Verbinden von zwei Körpergewebeteilen miteinander wieder zu entfernen, ohne eine mit dem Instrument hergestellte Verbindung zwischen den beiden Körpergewebeteilen zu überdehnen, was zu einer Gefährdung des Operationsergebnisses führen würde.

Es ist daher eine Aufgabe der vorliegenden Erfindung, ein chirurgisches Instrument der eingangs beschriebenen Art so zu verbessern, dass eine mechanische Belastung von insbesondere mittels eines Stromflusses erzeugten Körpergewebeteilverbindungen beim Entfernen des chirurgischen Instruments minimiert wird.

Diese Aufgabe wird durch das in Anspruch 1 spezifizierte chirurgische Instrument gelöst.

Die vorgeschlagene Weiterbildung eines chirurgischen Instruments der eingangs beschriebenen Art ermöglicht es insbesondere, eine Länge der von der Elektrode in der Annäherungsstellung definierten Umfangslinie zu verändern, nämlich beim Übergang in die Entfernungsstellung zu verkleinern. Dies gestattet es dann insbesondere, das zweite Werkzeugelement ebenfalls beim Übergang von der Annäherungsstellung in die Entfernungsstellung in seiner äußeren Erscheinung, insbesondere seiner Größe, derart zu ändern, dass es durch die hergestellte Verbindung, also die Verbindungsstelle, hindurch zurückgezogen werden kann, ohne die Verbindungsstelle zu verformen oder gar zu überdehnen. Durch das einfache Abklappen des zweiten Werkzeugelements, wie es beispielsweise aus der DE 20 2010 013 152 U1 bekannt ist, muss die Verbindungsstelle beim Zurückziehen des Instruments zumindest verformt werden. Eine schwache Dehnung kann ebenso nicht vollständig ausgeschlossen werden. Durch die Veränderung einer Länge der Umfangslinie der Elektrode beim Übergang von der Annäherungsstellung in die Entfernungsstellung wird insbesondere auch die vom zweiten Werkzeugelemente definierte effektive Fläche verringert, und dies sogar unabhängig von einer Winkelstellung des zweiten Werkzeugelements relativ zu einer insbesondere vom Schaft im Bereich der Werkzeugelemente definierten Längsachse. Mit dem chirurgischen Instrument lassen sich also Körpergewebeteile noch schonender als mit bekannten Instrumenten verbinden.

Günstig ist es, insbesondere auch bei einem chirurgischen Instrument der eingangs beschriebenen Art, wenn eine vom zweiten Werkzeugelement in der Entfernungsstellung definierte erste Werkzeugumfangslinie kürzer ist als eine vom zweiten Werkzeugelement in der Betriebsstellung definierte zweite Werkzeugumfangslinie. Mit einem zweiten Werkzeugelement, das seine Form oder äußere Kontur derart ändern kann, ist es möglich, das zweite Werkzeugelement durch die mit dem Instrument erzeugte Verbindung hindurch zu bewegen, ohne die Verbindungsstelle zu verformen oder zu überdehnen.

Die Handhabung des Instruments lässt sich insbesondere dadurch weiter verbessern, dass es eine Rückhalteeinrichtung zum Sichern des zweiten Werkzeugelements in der Betriebsstellung umfasst. Mit der Rückhalteeinrichtung kann also insbesondere sichergestellt werden, dass das Werkzeugelement nicht in unbeabsichtigter Weise von der Betriebsstellung in die Entfernungsstellung überführt werden kann.

Vorteilhaft ist es, wenn die Rückhalteeinrichtung und das zweite Werkzeugelement relativ zueinander bewegbar angeordnet sind. Beispielsweise kann so eine Relativbewegung der Rückhalteeinrichtung und des zweiten Werkzeugelements genutzt werden, um das Werkzeugelement von der Betriebsstellung in die Entfernungsstellung zu überführen.

Gemäß einer weiteren bevorzugten Ausführungsform der Erfindung kann vorgesehen sein, dass das Instrument einen Faltmechanismus zum Überführen des zweiten Werkzeugelements von der Betriebsstellung in die Entfernungsstellung und/oder umgekehrt umfasst. Mit dem Faltmechanismus kann das zweite Werkzeugelement optional insbesondere derart in seiner Form und/oder Gestalt geändert werden, dass die Annäherungsumfangslinie in die Rückzugsumfangslinie verkürzbar und/oder die zweite Werkzeugumfangslinie in die erste Werkzeugumfangslinie verkürzbar ist.

Besonders einfach lässt sich der Faltmechanismus betätigen, wenn er ein erstes Kraftübertragungselement zum Übertragen einer Betätigungskraft auf die Rückhalteeinrichtung zum Überführen derselben von der Betriebsstellung in die Entfernungsstellung und/oder umgekehrt umfasst. Mit dem ersten Kraftübertragungselement ist es somit insbesondere gezielt möglich, beispielsweise rein mechanisch, die Rückhalteeinrichtung zu betätigen oder zu bewegen, um diese beziehungsweise das Instrument von der Betriebs- oder Annäherungsstellung in die Entfernungsstellung und/oder umgekehrt zu überführen.

Vorteilhafterweise sind die Rückhalteeinrichtung und ein zweites Kraftübertragungselement zum Übertragen einer Betätigungskraft auf das zweite Werkzeugelement zum Überführen desselben von der Betriebsstellung in die Annäherungsstellung und/oder umgekehrt relativ zueinander bewegbar angeordnet. Auf diese Weise ist es insbesondere möglich, den Faltmechanismus und das zweite Werkzeugelement in definierter Weise und unabhängig voneinander zu betätigen.

Besonders einfach wird der konstruktive Aufbau des chirurgischen Instruments, wenn das zweite Kraftübertragungselement und die Rückhalteeinrichtung relativ zueinander verschieb- und/oder verdreh- und/oder verschraubbar ausgebildet sind.

Günstig ist es, wenn die Rückhalteeinrichtung und/oder das erste und/oder das zweite Kraftübertragungselement relativ zum Schaft bewegbar angeordnet sind. Damit kann beispielsweise ein Operateur das Instrument am Schaft halten und in gezielter und gewünschter Weise wahlweise die Rückhalteeinrichtung oder das erste oder das zweite Kraftübertragungsglied betätigen, beispielsweise um Körpergewebe miteinander zu verbinden oder das Instrument in definierter Weise aus dem Körper eines Patienten zu entfernen.

Gemäß einer weiteren bevorzugten Ausführungsform der Erfindung kann das Instrument einen mit dem Faltmechanismus und/oder dem zweiten Kraftübertragungselement und/oder der Rückhalteeinrichtung gekoppelten Betätigungsmechanismus zum Betätigen des Faltmechanismus und/oder zum Bewegen des zweiten Kraftübertragungsglieds und/oder der Rückhalteeinrichtung relativ zum Schaft umfassen. Der Betätigungsmechanismus kann insbesondere ein oder mehrere Betätigungselemente oder Betätigungsglieder umfassen, die direkt oder indirekt mit dem Faltmechanismus, dem zweiten Kraftübertragungselement oder der Rückhalteeinrichtung gekoppelt sind oder mit diesen zusammenwirken, um diese vorzugsweise wahlweise zu betätigen.

Vorzugsweise umfasst das zweite Werkzeugelement mindestens zwei Werkzeugelementglieder, welche mit der zweiten Elektrode gekoppelt sind oder einen Teil derselben tragen. Vorzugsweise ist eine Mehrzahl von Werkzeugelementgliedern vorgesehen, die insbesondere identisch ausgebildet sein können. Vorteilhaft ist es, wenn das zweite Werkzeugelement rotationssymmetrisch bezogen auf eine von diesem definierte Längsachse ausgebildet ist. Insbesondere können die Werkzeugelementglieder relativ zueinander bewegbar angeordnet oder ausgebildet sein, wodurch sich auf einfache Weise eine äußere Kontur oder Form des zweiten Werkzeugelements ändern lässt.

Günstigerweise sind die mindestens zwei Werkzeugelementglieder in radialer Richtung bezogen auf eine vom zweiten Werkzeugelement definierte Längsachse bewegbar angeordnet oder ausgebildet. Beispielsweise kann es günstig sein, wenn freie Enden der Werkzeugelementglieder beim Übergang von der Betriebsstellung in die Entfernungsstellung in Richtung auf die Längsachse hin bewegbar sind.

Erfindungsgemäß sind die mindestens zwei Werkzeugelementglieder in Form von mindestens teilweise in radialer Richtung abstehenden Arme ausgebildet. Derartige Werkzeugelementglieder lassen sich beispielsweise auf einfache Weise ausbilden durch Einschneiden eines schirm-, deckel oder hülsenförmig ausgebildeten zweiten Werkzeugelements.

Um eine möglichst stabile Anordnung zu erhalten, ist es günstig, wenn die mindestens zwei Werkzeugelementglieder am distalen Ende des zweiten Werkzeugelements angeordnet oder festgelegt sind und deren freie Enden in proximaler oder im Wesentlichen in proximaler Richtung weisen. Insbesondere können so die freien Enden der Werkzeugelementglieder mit Elektrodenabschnitten ausgestattet oder mit einer Elektrode gekoppelt sein, welche in proximaler Richtung weisen und mit einer in distaler Richtung weisenden Elektrode des ersten Werkzeugelements zusammenwirken können.

Die Herstellung insbesondere des zweiten Werkzeugelements wird besonders einfach, wenn die mindestens zwei Werkzeugelementglieder durch einen geradlinigen oder gekrümmten, sich in radialer oder im Wesentlichen in radialer Richtung erstreckenden Schlitz voneinander getrennt sind. Dies ermöglicht es, die Werkzeugelementglieder beispielsweise so weit aufeinander zu und insbesondere auch in Richtung auf die Längsachse zu bewegen, wie der vorgesehene Schlitz hierfür Spielraum lässt. Mit anderen Worten ist es günstig, wenn der Schlitz eine endliche Breite aufweist, welche es ermöglicht, einen Abstand zwischen den Werkzeugelementgliedern in Umfangsrichtung beim Übergang von der Betriebsstellung in die Entfernungsstellung zu verringern.

Erfindungsgemäß ist die Elektrode des zweiten Werkzeugelements in Form eines Elektrodenrings ausgebildet, welcher einen variablen Umfang aufweist. Ein solcher Elektrodenring ermöglicht es, auf einfache Weise zu erreichen, dass die Annäherungsumfangslinie länger ist als die Rückzugsumfangslinie.

Erfindungsgemäß ist der Elektrodenring in radialer Richtung geschlitzt. Insbesondere können mehrere Schlitze vorgesehen sein. Ist nur ein Schlitz vorgesehen, können insbesondere freie Enden des Elektrodenrings aufeinander zu oder voneinander weg bewegt werden, um so eine Länge der von der Elektrode definierten Umfangslinie zu ändern. In gleicher Weise kann dies auch erreicht werden, wenn der Elektrodenring durch mehrere Schlitze in eine entsprechende Zahl von Elektrodenabschnitten unterteilt wird.

Gemäß der Erfindung ist der Elektrodenring hohl und definiert einen Elektrodenringkanal wobei im Elektrodenringkanal ein Elektrodenringausgleichselement bewegbar gehalten ist zum Verbinden freier Enden des geschlitzten Elektrodenrings. Das Elektrodenringausgleichselement ermöglicht es, unabhängig von einem Abstand freier Enden des Elektrodenrings, die durch einen Schlitz voneinander getrennt sind, eine durchgehende ringförmige Elektrode auszubilden. Diese kann dann insbesondere im Bereich des Schlitzes, also dort, wo das Elektrodenringausgleichselement insbesondere aus den freien Enden des Elektrodenrings hervorsteht, einen etwas kleineren Außendurchmesser aufweisen als der Elektrodenring selbst.

Günstig ist es, wenn der Elektrodenring mehrere, durch Schlitze voneinander getrennte Elektrodenringabschnitte umfasst, welche jeweils an einem freien Ende eines Werkzeugelementglieds angeordnet oder ausgebildet sind. Beispielsweise kann so bei einer Mehrzahl von Werkzeugelementgliedern eine entsprechende Zahl an Elektrodenringabschnitten ausgebildet sein. Diese können insbesondere in proximaler Richtung weisende Elektrodenflächenbereiche definieren, so dass die Elektrode insgesamt eine Mehrzahl von optional beabstandeten oder jeweils benachbarte Elektrodenringabschnitte berührenden Elektrodenringabschnitte umfasst. So lässt sich insbesondere auch eine Umfangslinie der Elektrode infolge einer Bewegung der Werkzeugelementglieder beim Übergang von der Betriebsstellung in die Entfernungsstellung in gewünschter Weise verkürzen.

Auf besonders einfache Weise kann ein durchgehender Elektrodenring ausgebildet werden, und zwar unabhängig davon, ob das zweite Werkzeugelement die Betriebsstellung oder die Entfernungsstellung einnimmt, wenn der Elektrodenring in sich geschlossen und aus einem elastischen oder dehnbaren Material hergestellt ist. Vorzugsweise ist das Material zur Ausbildung des Elektrodenrings elektrisch leitfähig. Es kann insbesondere Nitinol enthalten oder Nitinol sein, wodurch Elastizitäten erreichbar sind, die eine Änderung der durch die Elektrode definierten Umfangslinie um bis zu 8% zulassen.

Ferner kann es vorteilhaft sein, wenn das zweite Werkzeugelement eine Vorspanneinrichtung umfasst zum vorspannenden Halten des zweiten Werkzeugelements gegen die Rückhalteeinrichtung in der Betriebsstellung. Die Vorspanneinrichtung kann einerseits eine Stabilität des zweiten Werkzeugelements verbessern. Andererseits ermöglicht sie es auch, beispielsweise bei einer Bewegung der Rückhalteeinrichtung relativ zum zweiten Werkzeugelement ,das Werkzeugelement automatisch von der Betriebsstellung in die Entfernungsstellung zu überführen.

Der konstruktive Aufbau der Vorspanneinrichtung lässt sich insbesondere dadurch vereinfachen, dass sie mindestens ein Vorspannglied umfasst. Es können auch zwei oder mehr Vorspannglieder vorgesehen sein.

Besonders einfach herstellen lässt sich das chirurgische Instrument, wenn das mindestens eine Vorspannglied in Form eines mindestens teilweise federelastischen Elements ausgebildet ist. Denkbar sind hier insbesondere Blatt- oder Schraubenfedern. In Frage kommen insbesondere auch in einem gewissen Rahmen elastische oder federelastische Kunststoffelemente.

Besonders einfach und kompakt ausbilden lässt sich das chirurgische Instrument, wenn mindestens ein Teil der mindestens zwei Werkzeugelementglieder ein Vorspannglied umfasst oder bildet. Es ist also insbesondere denkbar, dass die Werkzeugelementglieder selbst die Vorspannglieder bilden. So kann das zweite Werkzeugelement beispielsweise aus einem oder mehreren Kunststoffteilen ausgebildet sein. Werden die Werkzeugelementglieder in Form von voneinander getrennten Kunststoffarmen ausgebildet, weisen diese von Haus aus eine gewisse Elastizität auf. Werden sie insbesondere beim Übergang von der Entfernungsstellung in die Betriebsstellung aufgespreizt, beispielsweise durch die Rückhalteeinrichtung, so halten sie das zweite Werkzeugelement unter Vorspannung gegen die Rückhalteeinrichtung. Wird diese relativ zum zweiten Werkzeugelement bewegt, so dass die Werkzeugelementglieder freigegeben werden, können diese in ihre Ausgangsstellung zurückgehen und das zweite Werkzeugelement somit automatisch von der Betriebsstellung in die Entfernungsstellung überführt werden.

Gemäß einer weiteren bevorzugten Ausführungsform der Erfindung kann vorgesehen sein, dass die Rückhalteeinrichtung einen in radialer Richtung von einer Längsachse des zweiten Werkzeugelements weg weisend wirkenden Anschlag umfasst, an welchem die mindestens zwei Werkzeugelementglieder oder die Elektrode des zweiten Werkzeugelements in der Betriebsstellung direkt oder indirekt anliegen. Durch diesen Anschlag können die Werkzeugelementglieder beispielsweise in der zuvor beschriebenen, ausgelenkten Stellung gehalten werden, aus der sie nach Freigabe durch den Anschlag wieder in ihre Grundstellung übergehen können, also insbesondere von der Betriebsstellung in die Entfernungsstellung.

Besonders einfach wird die Ausgestaltung der Rückhalteeinrichtung, wenn diese ein ringförmiges oder im Wesentlichen ringförmiges Anschlagglied umfasst, welches die Längsachse umgibt und den Anschlag bildet oder umfasst. Das Anschlagglied kann also insbesondere in Form eines Rings oder Hülsenabschnitts ausgebildet sein, welcher eine in radialer Richtung von der Längsachse weg weisende Anschlagfläche umfasst, an welcher insbesondere die Werkzeugelementglieder oder freie Enden derselben in der Betriebsstellung direkt oder indirekt anliegen können.

Vorteilhaft ist es, wenn das Anschlagglied parallel zur Längsachse verschiebbar gehalten ist und die mindestens zwei Werkzeugelementglieder oder die Elektrode des zweiten Werkzeugelements beim Übergang von der Betriebsstellung in die Entfernungsstellung freigibt. Insbesondere wenn die Werkzeugelemente in der Betriebsstellung gegen das Anschlagglied gehalten sind, können sie bei einer Bewegung des Anschlagglieds relativ zu ihnen in eine Grundstellung, insbesondere die Entfernungsstellung, übergehen, wenn das Anschlagglied nicht mehr als Anschlag wirken kann, also wenn es die Werkzeugelementglieder freigibt.

Günstig ist es, wenn das chirurgische Instrument ein Schneidelement zum Durchtrennen von Körpergewebe umfasst. Beispielsweise kann so überstehendes Gewebe im Bereich einer mit dem Instrument erzeugten Verbindungsstelle zwischen zwei Körpergewebeteilen abgetrennt werden.

Vorzugsweise ist das Schneidelement in Form einer die Längsachse umgebenden Ringschneide ausgebildet. Mit einer solchen Ringschneide kann eine mit dem Instrument erzeugte ringförmige Gewebeverbindungsstelle in gewünschter Weise präpariert werden. Das Schneidelement kann insbesondere als mechanisches Schneidelement mit einer geschärften Schneidkante oder als HF-Schneidelement ausgebildet sein.

Das Instrument lässt sich besonders kompakt ausbilden, wenn die Rückhalteeinrichtung das Schneidelement umfasst oder trägt. Dies ermöglicht es insbesondere, das Schneidelement zu bewegen, wenn das zweite Werkzeugelement die Annäherungsstellung einnimmt und zwei miteinander zu verbindende Körpergewebeteile durch die aufeinander zu weisenden Elektroden der ersten und zweiten Werkzeugelemente miteinander verbunden wurden. Wird das Schneidelement dann bewegt, kann insbesondere auch die Rückhalteeinrichtung derart mitbewegt werden, dass sie einen Übergang des zweiten Werkzeugelements von der Betriebsstellung in die Entfernungsstellung ermöglicht. Dies kann somit insbesondere in einem Schritt mit dem Durchtrennen überstehenden Gewebes im Bereich der Gewebeverbindungsstelle erfolgen.

Um überstehendes Gewebe im Bereich der Verbindungsstelle beim Entfernen des Instruments abzutrennen, ist es vorteilhaft, wenn eine Schneidkante des Schneidelements in proximaler Richtung weist.

Die Handhabbarkeit des chirurgischen Instruments lässt sich auf einfache Weise dadurch verbessern, dass der Betätigungsmechanismus mindestens ein Betätigungsglied umfasst, welches an einem proximalen Ende oder in einem proximalen Endbereich des Instruments angeordnet oder bewegbar gehalten ist. Insbesondere können mehrere Betätigungsglieder vorgesehen sein, die mit dem zweiten Werkzeugelement beziehungsweise der Rückhalteeinrichtung gekoppelt sind, um wahlweise eine Bewegung derselben relativ zum Schaft des Instruments zu ermöglichen.

Die nachfolgende Beschreibung bevorzugter Ausführungsformen der Erfindung dient im Zusammenhang mit den Zeichnungen der näheren Erläuterung. Ausführungsformen der Erfindung sind in den Figuren 1-9b dargestellt. Es zeigen:
- Figur 1:: eine schematische, perspektivische Gesamtansicht eines chirurgischen Instruments zum Verbinden von Körpergewebe;
- Figur 2:: eine teilweise geschnittene Ansicht eines distalen Endes des in Figur 1 dargestellten Instruments beim Herstellen einer End-zu-Seit-Anastomose;
- Figur 3:: eine vergrößerte Ausschnittsansicht der Anordnung aus Figur 2;
- Figur 4:: eine teilweise durchbrochene Seitenansicht des Instruments vor dem Verbinden von Körpergewebe;
- Figur 5:: eine Ansicht des Instruments ähnlich der Darstellung in Figur 3 beim Verbinden von Körpergewebe;
- Figur 6:: eine Ansicht ähnlich der Darstellung in Figur 5 beim Abtrennen überstehenden Körpergewebes im Bereich der Verbindungsstellung;
- Figur 7:: eine Ansicht ähnlich der Darstellung in Figur 4 beim Überführen des Instruments von der Betriebsstellung in die Entfernungsstellung;
- Figur 8:: eine Ansicht ähnlich der Darstellung in Figur 6 nach Überführen des zweiten Werkzeugelements von der Betriebsstellung in die Entfernungsstellung ;
- Figur 9a:: eine perspektivische Ansicht des zweiten Werkzeugelements mit Rückhalteeinrichtung von unten;
- Figur 9b:: eine Ansicht ähnlich der Darstellung in Figur 9a ohne Rückhalteeinrichtung;
- Figur 10a:: eine perspektivische Seitenansicht eines weiteren Ausführungsbeispiels eines zweiten Werkzeugelements;
- Figur 10b:: eine Teilexplosionsdarstellung der Anordnung aus Figur 10a;
- Figur 10c:: eine Explosionsdarstellung der Anordnung aus Figur 10a;
- Figur 11:: eine Längsschnittansicht des in Figur 10a dargestellten zweiten Werkzeugelements in der Betriebs- und Annäherungsstellung;
- Figur 12:: eine Ansicht ähnlich der Darstellung in Figur 11, jedoch in der Entfernungsstellung;
- Figur 13:: eine Ansicht analog Figur 11 eines weiteren Ausführungsbeispiels eines zweiten Werkzeugelements; und
- Figur 14:: eine perspektivische Ansicht eines weiteren Ausführungsbeispiels eines zweiten Werkzeugelements.

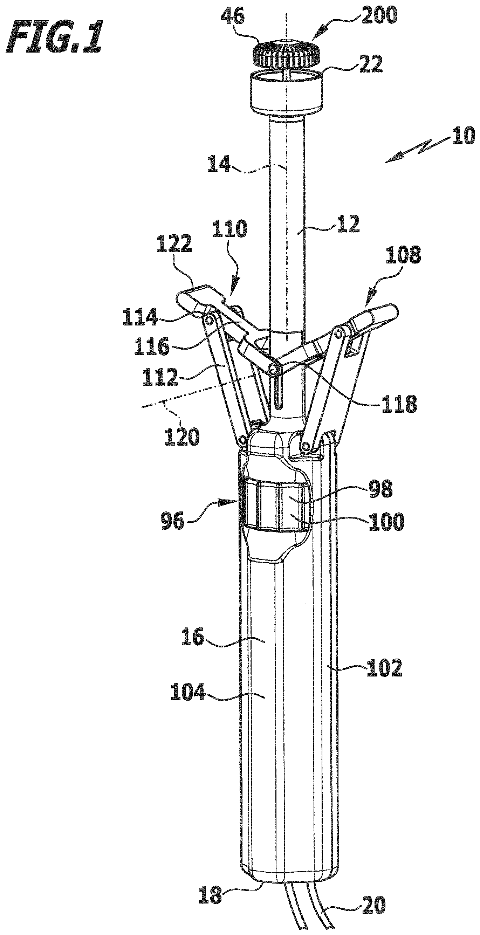

In Figur 1 ist beispielhaft ein chirurgisches Instrument 10 zum Verbinden von Körpergewebe dargestellt. Es umfasst einen langgestreckten Schaft 12, welcher eine Längsachse 14 definiert. Proximalseitig des Schafts 12 schließt sich an diesen ein schaftförmiger Griffbereich 16 an, welcher sich etwa über die Hälfte einer Gesamtlänge des Instruments 10 erstreckt. Aus einem proximalen Ende 18 des Griffbereichs 16 ragt eine Anschlussleitung 20 heraus, mit welcher das Instrument 10 mit einer in den Figuren nicht dargestellten Stromversorgung, beispielsweise einem HF-Generator, verbunden werden kann.

Der Schaft 12 erweitert sich zu einem distalen Ende 22 hin im Außendurchmesser im Wesentlichen einstufig, so dass ein ausgehend vom Ende 22 kurzer, zylindrischer Aufnahmeraum 24 ausgebildet wird, welcher in distaler Richtung weisend offen ist. Ausgehend vom Ende 22 ist in den Aufnahmeraum 24 an eine Innenwand 26 desselben angrenzend eine hülsenförmige Elektrode 28 eingesetzt, welche eine in Richtung auf die Längsachse 14 geneigte Elektrodenfläche 30 definiert. Die Elektrode 28 ist in nicht näher dargestellter Weise elektrisch leitend mit der Anschlussleitung 20 verbunden.

Konzentrisch zum Schaft 12 ist in diesem ein Innenschaft 32 angeordnet, welcher sich etwa bis zum proximalen Ende des Aufnahmeraums 24 erstreckt. Er definiert einen Kanal 34. Eine Abdeckung 36 kleidet den Aufnahmeraum 24 proximalseitig aus und lässt lediglich den Kanal 34 offen.

In dem eine Führung bildenden Innenschaft 32 ist längsverschiebblich ein stabförmiges erstes Kraftübertragungsglied 38 geführt, welches von einem zweiten, hülsenförmigen Kraftübertragungsglied 40 umgeben ist. Die Kraftübertragungsglieder 38 und 40 sind relativ zueinander parallel zur Längsachse 14 bewegbar.

Ein erstes Werkzeugelement 48 des Instruments 10 wird durch das Ende 22 mit der Elektrode 28 gebildet. Ein distales Ende 42 des ersten Kraftübertragungsglieds 38 greift formschlüssig in eine in proximaler Richtung geöffnete Ausnehmung 44 eines zweiten Werkzeugselements 46 ein.

Das zweite Werkzeugelement 46 ist in Form einer Art Deckel ausgebildet, welcher eine schwach konvex in distaler Richtung weisend gekrümmte Endfläche 50 mit einer stumpfen, abgerundeten Spitze 52 aufweist. Die Endfläche 50 geht, beabstandet von der Längsachse 14, in eine ringförmige Seitenwand 54 über, welche, in einer Betriebs- und Annäherungsstellung des Instruments 10, konzentrisch zur Längsachse 14 ausgebildet oder ausgerichtet ist. Eine in proximaler Richtung weisende Stirnfläche 56 der Seitenwand 54 ist konkav gekrümmt und dient zur Aufnahme und Befestigung einer Elektrode 58 des zweiten Werkzeugelements 46, die in Form eines Elektrodenrings 60 ausgebildet ist. Der Elektrodenring 60 ist hohl und definiert einen Elektrodenringkanal 62. Der Elektrodenring 60 ist in sich nicht geschlossen ausgebildet, sondern durch einen Schlitz 64 aufgetrennt, so dass aufeinander zu weisende Enden 66 und 68 etwas voneinander beabstandet sind. In den Elektrodenringkanal 62 ist ein Elektrodenringausgleichselement 70 eingesetzt, welches die beiden Enden 66 und 68 des geschlitzten Elektrodenrings 60 miteinander verbindet. Somit wird die insgesamt in sich geschlossene ringförmige Elektrode 58 ausgebildet, die zwei unterschiedliche Außendurchmesser aufweist, nämlich einen größeren, der definiert wird durch einen Außendurchmesser des Elektrodenrings 60, und einen kleineren Außendurchmesser zwischen den Enden 66 und 68, der definiert wird durch einen Außendurchmesser des Elektrodenringausgleichselements 70.

Das zweite Werkzeugelement 46 ist ausgehend von der Stirnwand 54 mit einer Mehrzahl von radialen Schlitzen 72 versehen, die gleichmäßig über einen Umfang des zweiten Werkzeugelements 46 verteilt sind. Auf diese Weise werden an der Spitze 52 zusammengehaltene, sich von dieser in radialer Richtung und längs der Seitenwand 54 in proximaler Richtung erstreckende Werkzeugelementglieder 74 ausgebildet, die im Wesentlichen die Form eines Arms aufweisen. Dadurch, dass das zweite Werkzeugelement 46 aus einem Kunststoff hergestellt ist, bilden die einen Teil der Endfläche 50 bildenden Abschnitte 76 Vorspannglieder 78 einer insgesamt mit dem Bezugszeichen 80 bezeichneten Vorspanneinrichtung 80. Diese dient dem Zweck, das zweite Werkzeugelement 46 vorspannend gegen eine insgesamt mit dem Bezugszeichen 82 bezeichnete Rückhalteeinrichtung zu halten.

Die Rückhalteeinrichtung 82 umfasst eine sich im Wesentlichen quer zur Längsachse 14 erstreckende Rückhaltescheibe 84, welche fest mit dem zweiten Kraftübertragungsglied 40 verbunden ist. Die Rückhaltescheibe 84 definiert einen maximalen Außendurchmesser 86 und ist mit einem ringförmigen, hülsenartigen Schneidelement 88 bestückt, welches die Längsachse 14 konzentrisch umgibt und eine in proximaler Richtung weisende Schneidkante 90 aufweist, die in sich geschlossen ist. Das Schneidelement 88 bildet somit einen Teil der Rückhalteeinrichtung 84. Die Rückhaltescheibe 84 und das Schneidelement 88 bilden zusammen ein Anschlagglied 87 aus, weiches einen Anschlag für die Werkzeugelementglieder 74 definiert, welcher in radialer Richtung von der Längsachse 14 weg wirkt. Insgesamt wird so ein Faltmechanismus 200 ausgebildet zum quasi Öffnen und Schließen des zweiten Werkzeugelements 46 ähnlich einem Regenschirm.

In einer Betriebsstellung des Instruments 10, wie sie schematisch in den Figuren 1 bis 5 dargestellt ist, sind die Werkzeugelementglieder 74 etwas in radialer Richtung aufgespreizt, so dass die Rückhalteeinrichtung 82 von proximal her kommend in das Werkzeugelement 46 vollständig eingeführt ist. Die Schneidkante 90 ist dabei etwas in distaler Richtung gegenüber der Elektrode 58 zurückversetzt, so dass das am weitesten vorspringende Teil des zweiten Werkzeugelements 46 die Elektrode 58 ist. Innenflächen 92 der Werkzeugelementglieder 74, die damit einen Teil der Seitenwand 54 bilden, sind aufgrund der Aufspreizung vorspannend gegen die Rückhalteeinrichtung 82 gehalten, nämlich gegen das Schneidelement 88 und die Rückhaltescheibe 84. Eine Bewegung freier Enden 94 der Werkzeugelementglieder 74 in Richtung auf die Längsachse 14 hin wird also durch die Rückhalteeinrichtung 82 verhindert.

Das Instrument 10 umfasst ferner einen Betätigungsmechanismus 96 mit einem ersten Betätigungselement 98 in Form eines Schraubrads 100, welches die Längsachse 14 konzentrisch umgibt und in einem vom Griffbereich 16 definierten Gehäuse um die Längsachse 14 rotierbar gelagert ist. Das Schraubrad 100 steht in Wirkverbindung mit einem im Außendurchmesser verdickten Abschnitt 104 des ersten Kraftübertragungsglieds 38, welcher ein Schraubengewinde 106 trägt. Dies ermöglicht es durch Rotation des Schraubrads 100 um die Längsachse 14 das erste Kraftübertragungsglied 38 in distaler beziehungsweise in proximaler Richtung zu bewegen. Eine Bewegung in distaler Richtung vergrößert einen Abstand zwischen den Elektroden 28 und 58, eine Bewegung des ersten Kraftübertragungsglieds in proximaler Richtung bewegt die Elektrode 58 in Richtung auf die Elektrode 28 hin.

Der Betätigungsmechanismus 96 umfasst ferner ein zweites Betätigungselement 108, welches in Form einer symmetrisch ausgebildeten Lenkeranordnung 110 ausgebildet. Zwei erste Lenker 112 sind seitlich versetzt zur Längsachse 14 um quer zu dieser und parallel zueinander verlaufende Schwenkachsen am Gehäuse 102 verschwenkbar gelagert. Freie Enden 114 der Lenker 112 sind wiederum verschwenkbar an jeweils einem weiteren Lenker 116 gelagert. Zwei freie Enden der Lenker 116 sind miteinander sowie dem zweiten Kraftübertragungsglied 40 verschwenkbar gekoppelt um eine die Längsachse 14 senkrecht schneidende Schwenkachse 120. Etwas versetzt von den anderen freien Enden 122 hin sind die Enden 114 mit dem Lenker 116 um parallel zur Schwenkachse 120 verlaufende Schwenkachsen verschwenkbar gelagert. Freie, von der Längsachse weg weisende Enden 122 der Lenker 116 bilden Betätigungsglieder zum Betätigen des zweiten Betätigungselements 108.

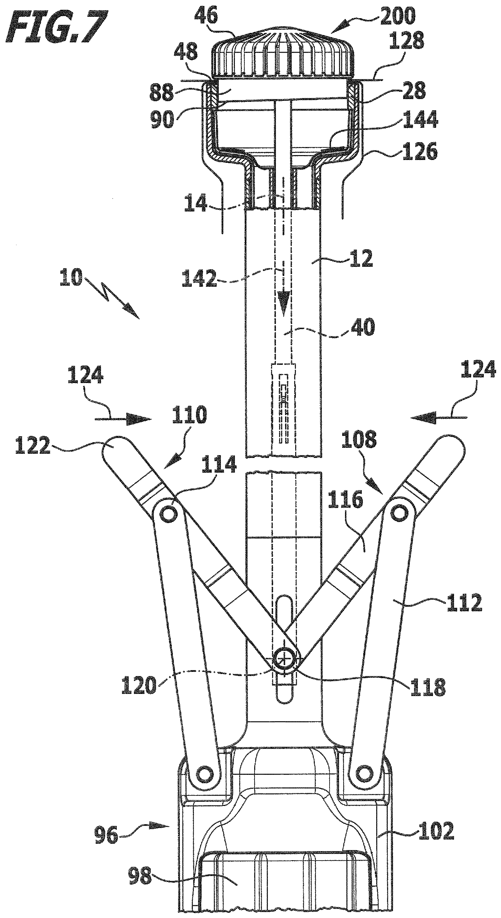

Werden die Enden 122 in Richtung der Pfeile 124, wie schematisch in Figur 7 dargestellt, aufeinander zu verschwenkt, wird das zweite Kraftübertragungsglied 40 relativ zum ersten Kraftübertragungsglied 38 in proximaler Richtung bewegt.

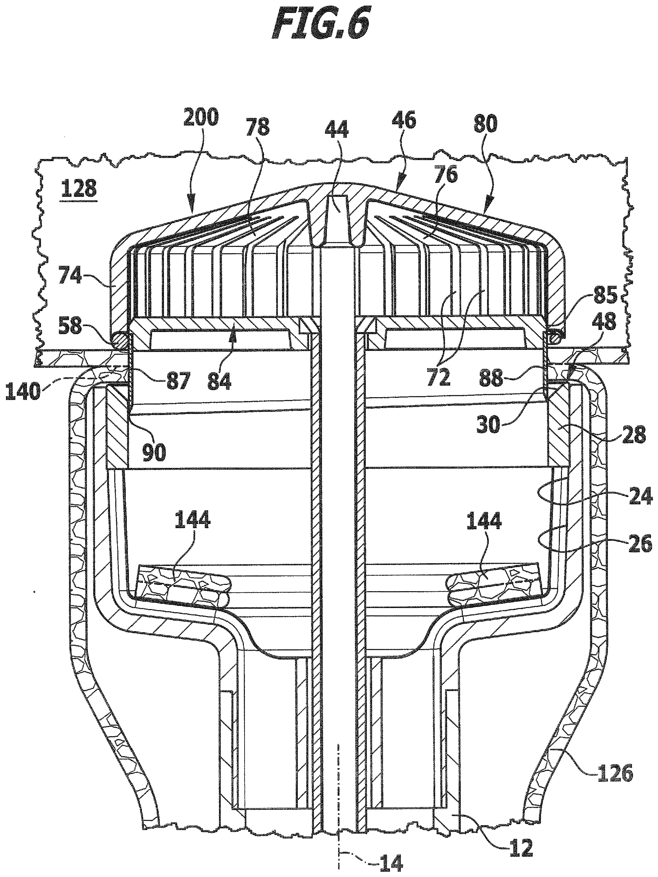

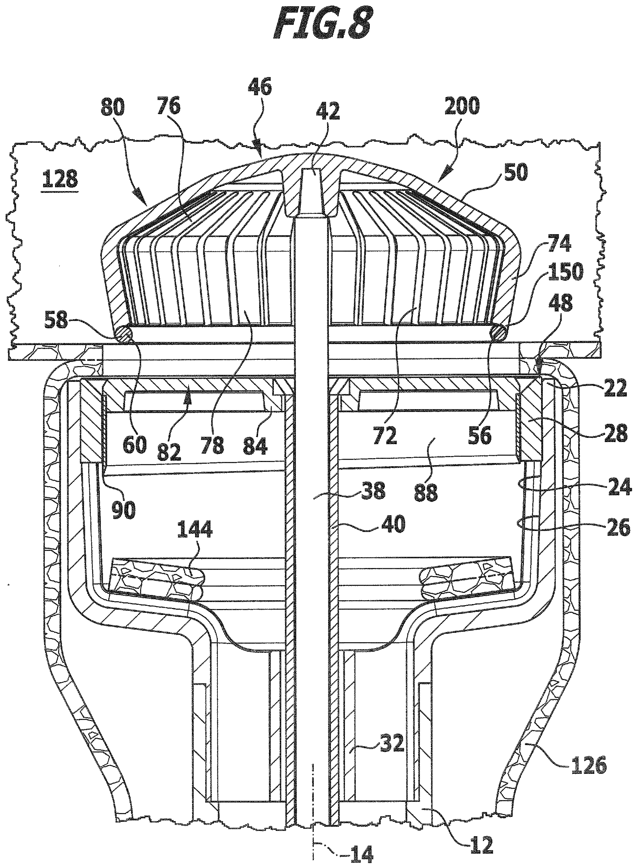

Die Funktionsweise des Instruments 10 wird nachfolgend in Verbindung mit den Figuren 1 bis 8 erläutert, und zwar rein beispielhaft in Verbindung mit der Herstellung einer End-zu-Seit-Anastomose.

Um ein erstes Gefäß 126 seitlich mit einem zweiten Gefäß 128 zu verbinden, wird das zweite Gefäß 126 durch eine kleine Inzision 130 eröffnet. Am ersten Gefäß 126 wird etwas von dessen Ende 132 entfernt eine Öffnung durch eine zweite Inzision 134 geschaffen. Das distale Ende des Instruments 10 wird mit dem zweiten Werkzeugelement 46 voran, welches zunächst die oben beschriebene Betriebsstellung einnimmt und durch die Rückhalteeinrichtung 82 in dieser vorspannend gehalten wird, durch die zweite Inzision 134 in das erste Gefäß 126 eingeführt, bis das zweite Werkzeugelement 46 aus dem Ende 132 hervorsteht. Das Ende 132 wird nach innen über die Elektrode 28 gelegt.

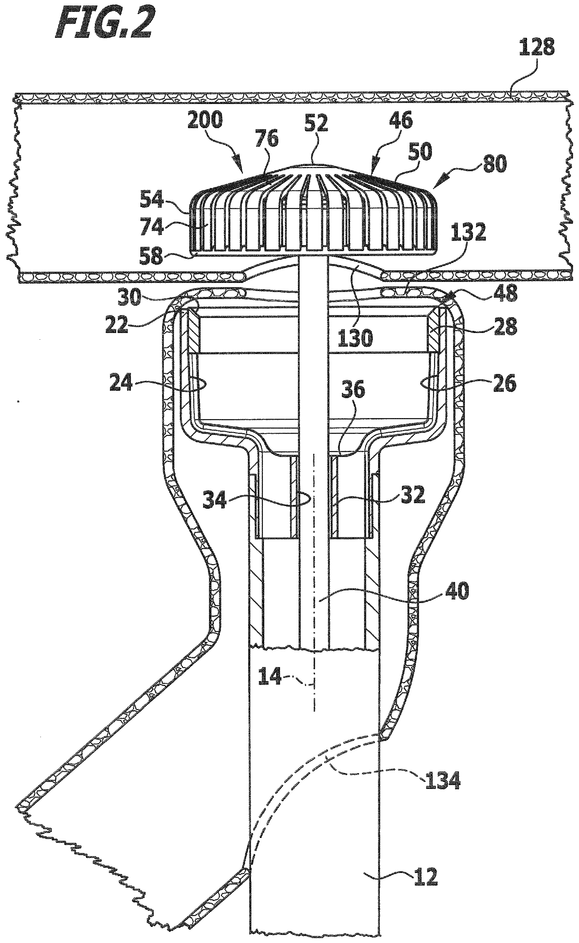

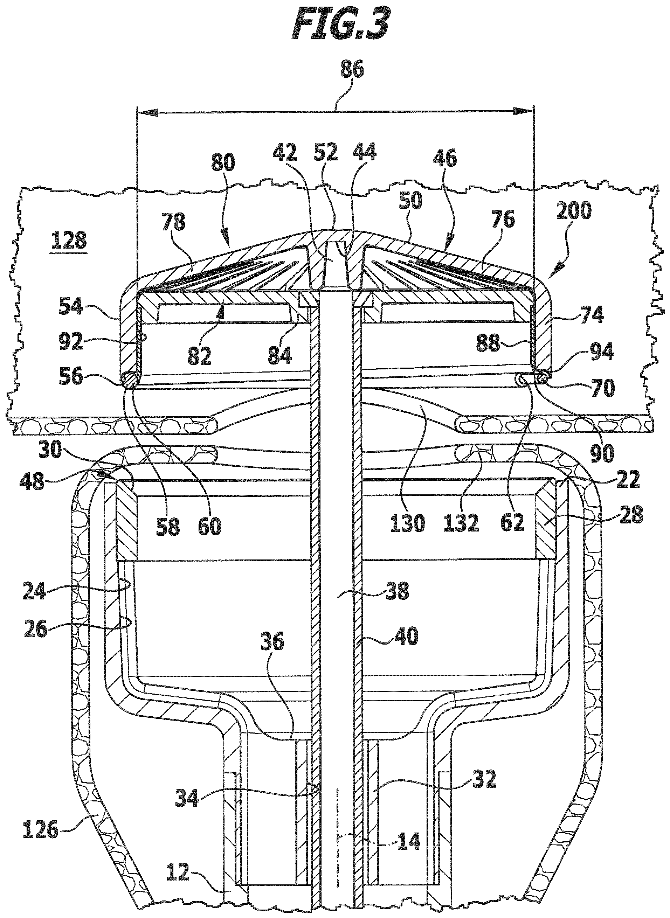

Im nächsten Schritt wird das Instrument 10, an welchem das erste Gefäß 126 in der beschriebenen Weise gehalten ist, mit dem zweiten Werkzeugelement 46 durch die erste Inzision 130 in das zweite Gefäß 128 eingeführt. Dies ist schematisch in Figur 2 dargestellt. Die Gefäße 126 und 128 werden so ausgerichtet, dass das Ende 132 des ersten Gefäßes 126 die erste Inzision 130 des zweiten Gefäßes 128 möglichst konzentrisch abdeckt. Somit liegen zwei Gewebeschichten übereinander, wie schematisch in Figur 3 dargestellt.

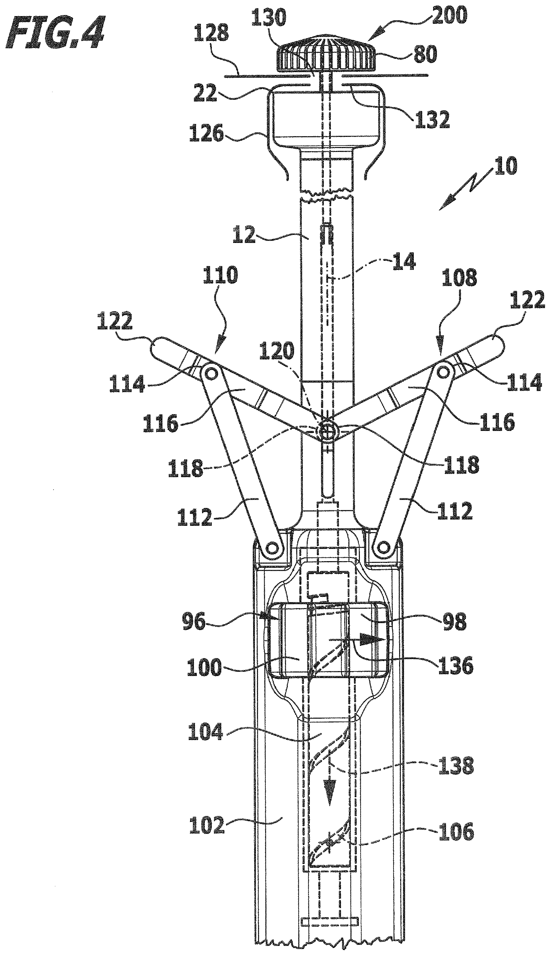

Zum Verbinden der übereinander liegenden Gewebeteile wird das Schraubrad 110, wie in Figur 4 schematisch dargestellt, in Richtung des Pfeils 136 gedreht, so dass das erste Kraftübertragungsglied 38 in Richtung des Pfeils 138 in proximaler Richtung bewegt wird.

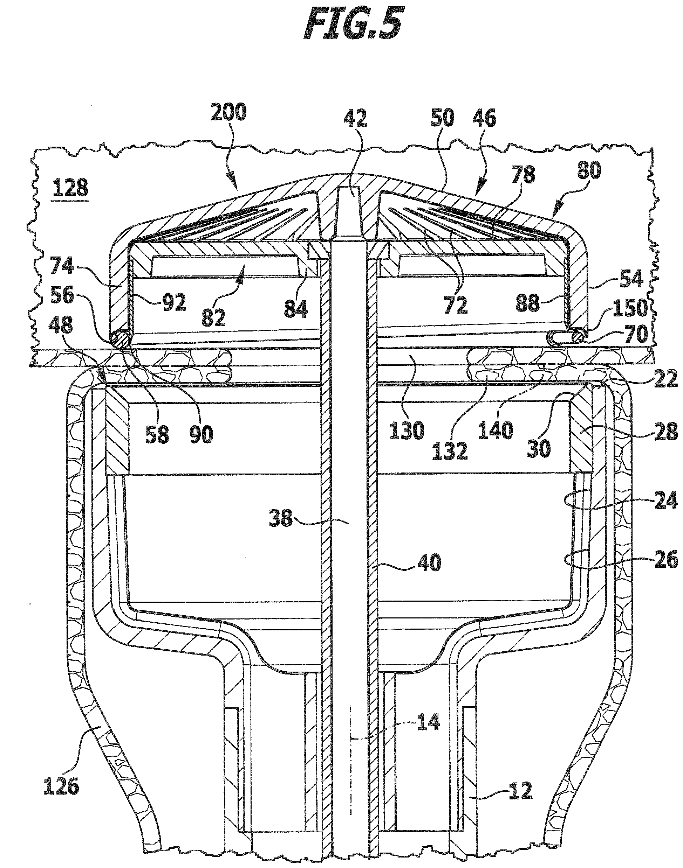

Sobald die Werkzeugelemente 46 und 48 eine Annäherungsstellung einnehmen, in welcher Sie einen minimalen Abstand voneinander definieren, wie schematisch in Figur 5 dargestellt, liegen die beiden Gewebeschichten aneinander an und werden durch Beaufschlagen der Elektroden 28 und 58 mit einem HF-Strom miteinander verbunden. Die Verbindung erfolgt quasi durch Verschweißen der Gefäße 126 und 128, da durch den gezielten Stromfluss über die Elektroden 28 und 58 in den Gefäßen 126 und 128 enthaltene Proteine so weit erwärmt werden, dass sie miteinander verkleben und eine dauerhafte und sofort belastbare Verbindung längs einer in sich geschlossenen ringförmigen Verbindungsstelle 140 zwischen den Elektroden 28 und 58 herstellen.

Sobald die Verbindung zwischen den Gefäßen 126 und 128 in definierter Weise erfolgt ist, kann das Instrument 10 wieder zurückgezogen werden. Hierfür wird das Instrument 10 zunächst von der Annäherungsstellung in die Entfernungsstellung überführt. Dies erfolgt durch Betätigen des zweiten Betätigungselements 108, und zwar indem die Enden 122 in Richtung der Pfeile 124 aufeinander zu verschwenkt werden. Durch die Bewegung des zweiten Kraftübertragungsglieds 40 in proximaler Richtung, also in Richtung des Pfeils 142, wie schematisch in Figur 7 dargestellt, wird auch die am zweiten Kraftübertragungsglied 40 gehaltene Rückhalteeinrichtung 82 in proximaler Richtung bewegt. Dies hat zur Folge, dass auch das Schneidelement 88 in proximaler Richtung bewegt wird, so dass die Schneidekante 90 die miteinander verbundenen Gefäße 126 und 128 benachbart der Verbindungsstelle 140 durchtrennen kann, so dass die in radialer Richtung auf die Längsachse 14 überstehenden Gewebeteile 144 abgetrennt und im Aufnahmeraum 24 aufgefangen werden. Während des Abtrennens der überstehenden Gewebeteile 144 behalten die Elektroden 28 und 58 ihren Abstand voneinander bei.

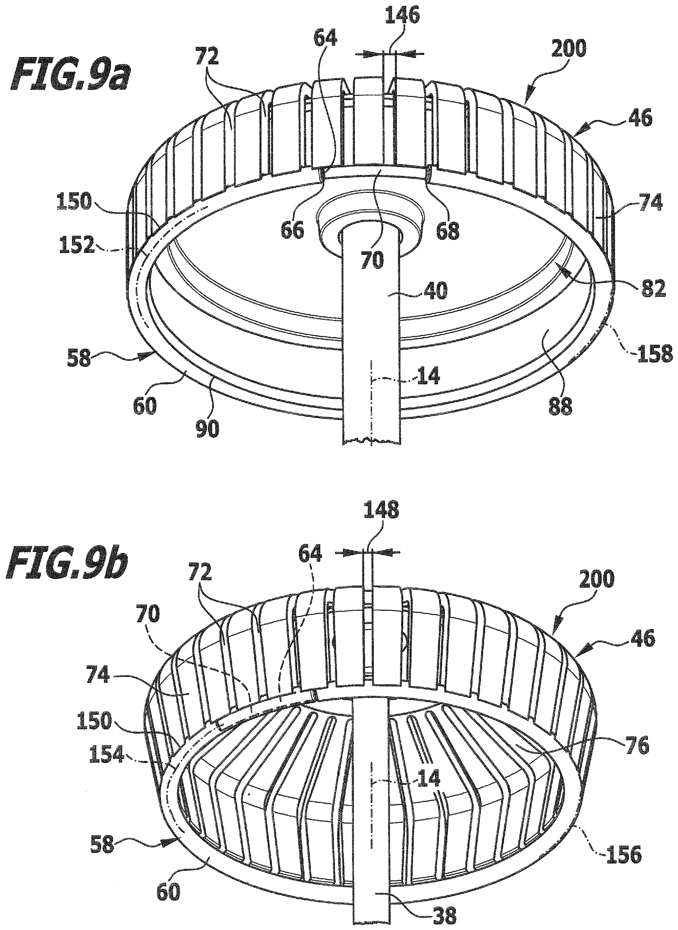

Sobald die Rückhaltescheibe 84 in proximaler Richtung den Elektrodenring 60 passiert hat, gibt die Rückhalteeinrichtung 82 das zweite Werkzeugelement 46, insbesondere freie Enden 150 der Werkzeugelementglieder 74, frei, so dass diese durch die Vorspannglieder 78 in radialer Richtung auf die Längsachse 14 hin bewegt werden können. Dabei verringert sich ein erster Abstand 146, welcher durch den Schlitz 72 in der Betriebsstellung definiert wird, wie schematisch in Figur 9a dargestellt, auf einen kleineren Abstand 148 in der Entfernungsstellung, wie er schematisch in Figur 9b dargestellt ist. Da so auch die freien Enden 150 der Werkzeugelementglieder 74 in Umfangsrichtung näher zusammenrücken können, verringert sich eine Umfangslinie der Elektrode 58 von einer Annäherungsumfangslinie 152 in der Betriebs- oder Annäherungsstellung, wie sie schematisch in Figur 9a eingezeichnet ist, in eine Rückzugsumfangslinie in der Entfernungsstellung, wie sie schematisch in Figur 9b dargestellt ist. Damit ist das zweite Werkzeugelement 46 von der Betriebsstellung, in welcher es in die Annäherungsstellung bringbar ist, in eine Entfernungsstellung bringbar, in welcher die von der Elektrode 58 definierte Rückzugsumfangslinie 154 kürzer ist, also die von der Elektrode 58 in der Annäherungsstellung definierte Annäherungsumfangslinie 152. Die Elektrode 58 ist somit auch in ihrem Umfang variabel. Ferner ist auch eine vom zweiten Werkzeugelement 46 in der Entfernungsstellung definierte erste Werkzeugumfangslinie 156 kürzer als eine vom zweiten Werkzeugelement 46 in der Betriebsstellung definierte zweite Werkzeugumfangslinie 158, wie dies schematisch in den Figuren 9a und 9b dargestellt ist.

Alternativ zum Elektrodenring 60 mit Elektrodenringausgleichselement 70 wäre es als nicht beanspruchte Alternative auch denkbar, die Elektrode 58 nicht durchgehend auszubilden, sondern lediglich die verbleibenden Stirnflächenbereiche an den distalen Enden 150 der Werkzeugelementglieder 74 elektrisch leitend auszubilden oder dort Elektrodenringabschnitte anzuordnen. Die Elektrode 58 würde dann aus einer Mehrzahl von Elektrodenringabschnitten bestehen, die durch die Schlitze 72 voneinander getrennt sind.

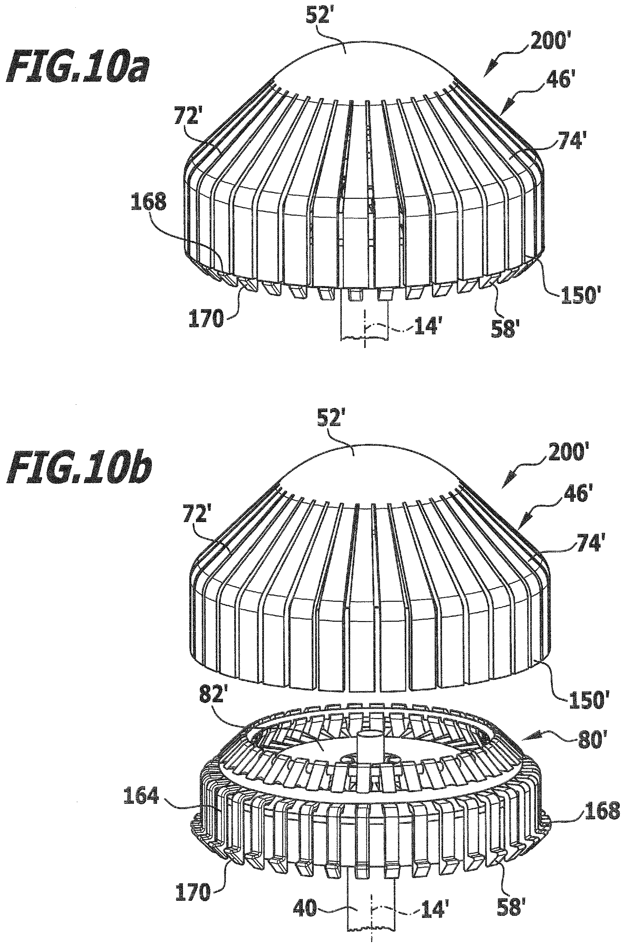

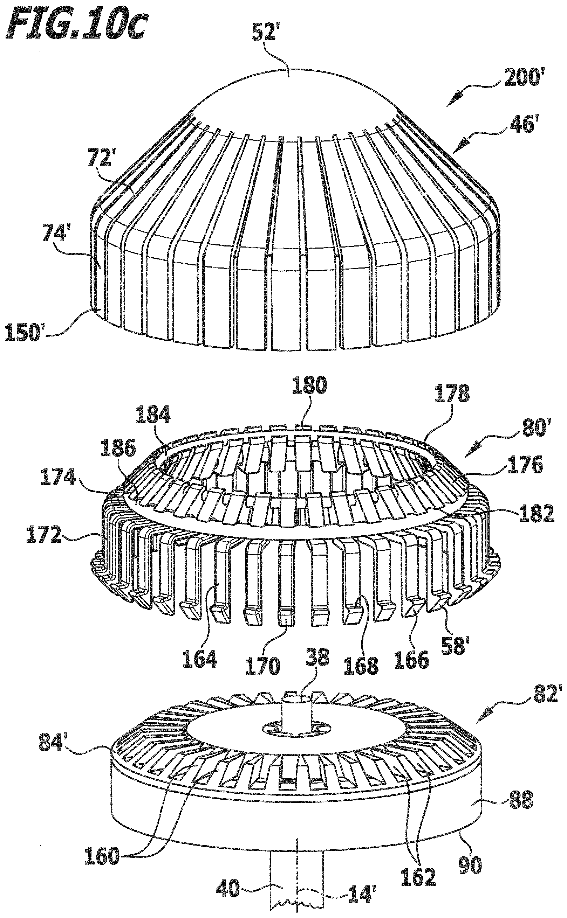

In den Figuren 10a bis 12 ist schematisch ein alternatives, nicht beanspruchtes Ausführungsbeispiel eines zweiten Werkzeugelements dargestellt und insgesamt mit dem Bezugszeichen 46' versehen. Das zweite Werkzeugelement 46' unterscheidet sich vom zweiten Werkzeugelement 46 im Wesentlichen durch den Aufbau der Elektrode 58' sowie der Vorspanneinrichtung 80'. Die Rückhalteeinrichtung 82' ist im Wesentlichen identisch ausgebildet mit der Rückhalteeinrichtung 82. Unterschiede bestehen lediglich in der Ausbildung der Rückhaltescheibe 84', welche eine Mehrzahl von in radialer Richtung orientierter und distaler Richtung weisender Rippen 160 umfasst. Zwischen jeweils zwei Rippen 160 wird eine in distaler Richtung geöffnete Aufnahme 162 ausgebildet, in welcher ein Elektrodenelement 164 in der Betriebs- oder Annäherungsstellung eintaucht. Jeweils durch eine Rippe 160 werden zwei Elektrodenelemente 164 in Umfangsrichtung voneinander beabstandet gehalten. Die Elektrodenelemente 164 sind im Wesentlichen L-förmig ausgebildet und weisen an ihrem distalen Ende einen in radialer Richtung von der Längsachse 14 weg abstehenden Vorsprung 166 auf, welcher eine in distaler Richtung weisende Anschlagfläche 168 für die Enden 150' der Werkzeugelementglieder 74' bildet. Der Vorsprung 166 definiert ferner eine Elektrodenfläche 170, die relativ zur Längsachse 14' nach außen geneigt ist.

In der Betriebsstellung erstreckt sich parallel zur Längsachse 14' und parallel zum Schneidelement 88 ein Abschnitt 172 des Werkzeugelementgliedes 74' . An den Abschnitt 172 schließt sich ein quer zu diesem und in radialer Richtung auf die Längsachse 14' hin gerichteter Abschnitt 174 an, welcher wiederum in einen um etwa 45° bezogen auf die Längsachse 14' geneigten und in Richtung auf die Spitze 52' hin weisenden Abschnitt 176 übergeht. Ein freies, vom Abschnitt 176 definiertes Ende 178 weist eine Aussparung 180 auf, die in Richtung auf die Spitze 52' hin geöffnet ist. Im Übergangsbereich zwischen den Abschnitten 174 und 176 ist eine weitere Aussparung 182 ausgebildet, die in radialer Richtung von der Längsachse 14 weg weisend geöffnet ist. in die Aussparung 180 der Elektrodenelemente 164 ist ein erster elastischer Ring 184 eingesetzt und kraft- und/oder formschlüssig gehalten. Ein zweiter elastischer, in der Betriebsstellung gedehnter Ring 186 ist in die Aussparungen 182 eingesetzt. Er dient dazu, auf die Elektrodenelemente 164 in der Betriebsstellung eine Zugkraft auszuüben, um die Elektrodenelemente 164 in Richtung auf die Längsachse 14 hin gegen die Rückhalteeinrichtung 82' vorspannend zu halten. Der Ring 186 bildet somit ein Vorspannglied 78'.

Die Elektrodenelemente 164 sind vorzugsweise aus einem elektrisch leitenden Material gebildet oder elektrisch leitend beschichtet. Der Ring 184 ist vorzugsweise ebenfalls aus einem elektrisch leitfähigen Material hergestellt, so dass alle Elektrodenelemente mittels des Elektrodenrings 184 elektrisch leitend miteinander verbunden sind. Der Ring 186 kann optional auch elektrisch leitend ausgebildet sein. Er kann jedoch beispielsweise auch aus einem elastischen Kunststoffring hergestellt sein.

Eine von der Schneidkante 90 definierte Schneidebene kann bezogen auf die Längsachse 14' geneigt sein. Dies kann nicht nur bei der Rückhalteeinrichtung 82' vorgesehen, sondern auch bei der Rückhalteeinrichtung 82 vorgesehen sein.

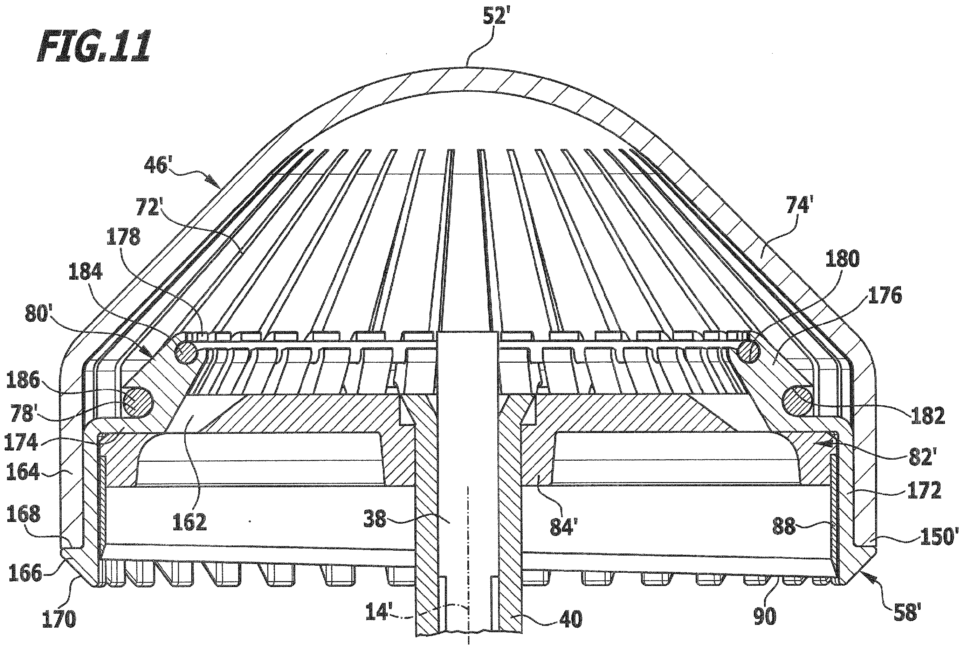

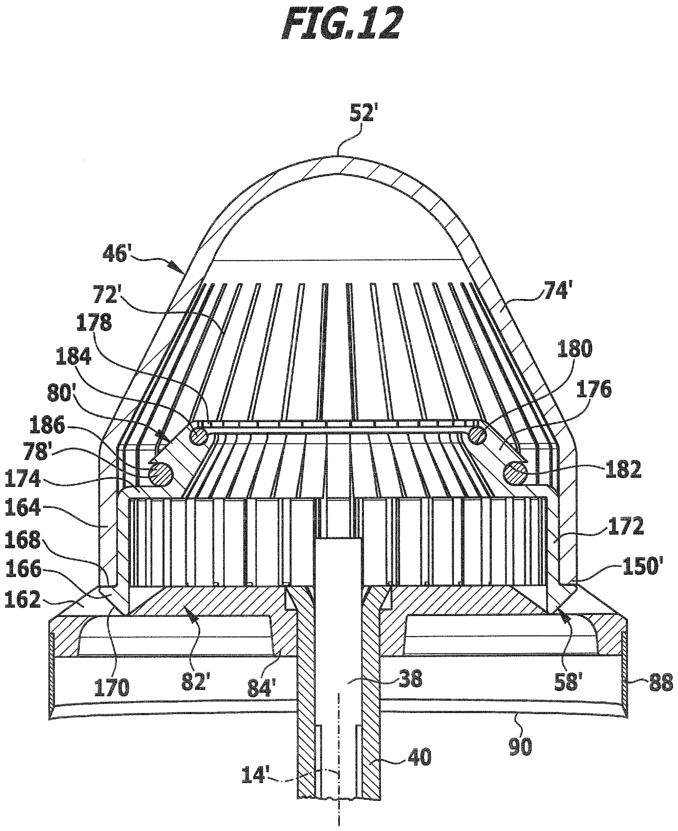

Die Funktionsweise des Instruments 10 ist unabhängig davon, ob ein zweites Werkzeugelement 46 oder ein zweites Werkzeugelement 46' vorgesehen ist, dieselbe. Wird das Instrument 10 mit dem zweiten Werkzeugelement 46', wie in Figur 11 schematisch in der Betriebsstellung dargestellt, in welcher das zweite Werkzeugelement 46' auch in die Annäherungsstellung bringbar ist, in die Entfernungsstellung überführt, wird in analoger Weise die Rückhalteeinrichtung 82' in proximaler Richtung bewegt, so dass der Ring 186 die Elektrodenelemente 164 in Richtung auf die Längsachse 14 hin bewegen kann. Dadurch wird erreicht, dass sowohl eine vom zweiten Werkzeugelement 46' definierte Werkzeugumfangslinie als auch eine von der Elektrode 58' definierte Umfangslinie in der Annäherungsstellung jeweils deutlich länger sind als in der Entfernungsstellung. Dies ergibt sich direkt aus einer Verringerung des Durchmessers des zweiten Werkzeugelements 46', der durch die freien Enden 150 der Werkzeugelementglieder 74' begrenzt wird, wie dies schematisch in den Figuren 11 und 12 dargestellt ist. Insgesamt wird so ein Faltmechanismus 200' ausgebildet zum quasi Öffnen und Schließen des zweiten Werkzeugelements 46' ähnlich einem Regenschirm.

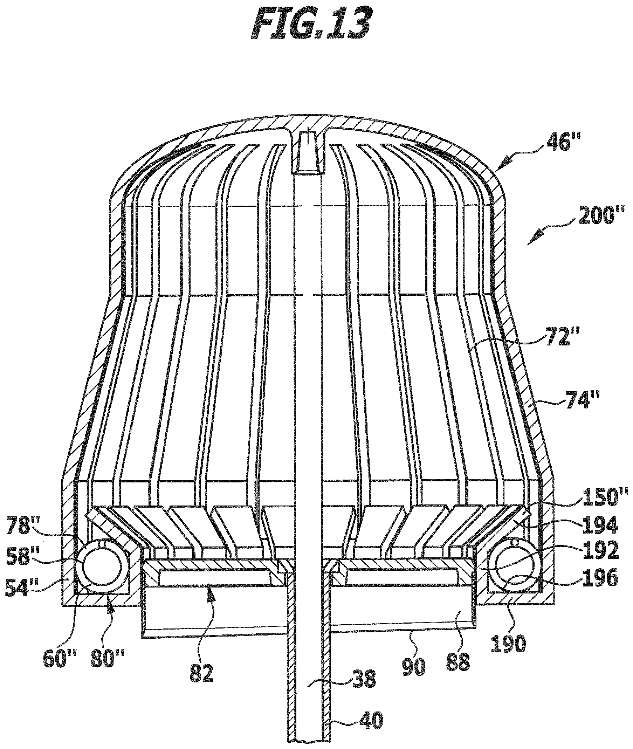

Ein weiteres nicht beanspruchtes alternatives Ausführungsbeispiel eines zweiten Werkzeugelements ist in Figur 13 schematisch dargestellt und insgesamt mit dem Bezugszeichen 46' versehen. Es unterscheidet sich vom zweiten Werkzeugelement 46 im Wesentlichen dadurch, dass freie Ende 150" der Werkzeugelementglieder 74" dreifach nach innen umgebogen beziehungsweise gekrümmt sind, so dass zunächst ein sich in radialer Richtung erstreckender Abschnitt 190 an die Seitenwand 54" ausgebildet wird, ein sich daran anschließender, parallel zur Längsachse 14 in distaler Richtung erstreckender Abschnitt 192 und ein in distaler und radialer Richtung geneigter Abschnitt 194, der gleichzeitig das freie Ende 150" bildet. Die Abschnitte 190, 192 und 194 definieren somit quasi eine Aufnahme 196 für einen Elektrodenring 60". Dieser kann beispielsweise in sich geschlossen ausgebildet sein und ganz oder teilweise aus Nitinol bestehen. Auf diese Weise weist er eine Elastizität von bis zu etwa 8% auf. Er bildet damit gleichzeitig das Vorspannglied 78" einer insgesamt mit dem Bezugszeichen 80" bezeichneten Vorspanneinrichtung.

Das zweite Werkzeugelement 46" ist in Figur 13 schematisch in der Betriebsstellung dargestellt. Die Abschnitte 192 liegen in der Betriebstellung an der Rückhalteeinrichtung 82 an, die identisch mit der im Zusammenhang mit den Figuren 1 bis 9b beschriebenen Rückhalteeinrichtung 82 ausgebildet. Wird diese in proximaler Richtung in der oben beschriebenen Weise durch die Bewegen des zweiten Kraftübertragungsglieds 40 in proximaler Richtung verschoben, werden die Werkzeugelementglieder 74" freigegeben und der Elektrodenring 60" kann sich zusammenziehen, um eine von der Elektrode 58" definierte Elektrodenlinie beim Übergang von der Annäherungsstellung in die Entfernungsstellung zu verkürzen. Insgesamt wird so ein Faltmechanismus 200" ausgebildet zum quasi Öffnen und Schließen des zweiten Werkzeugelements 46" ähnlich einem Regenschirm.

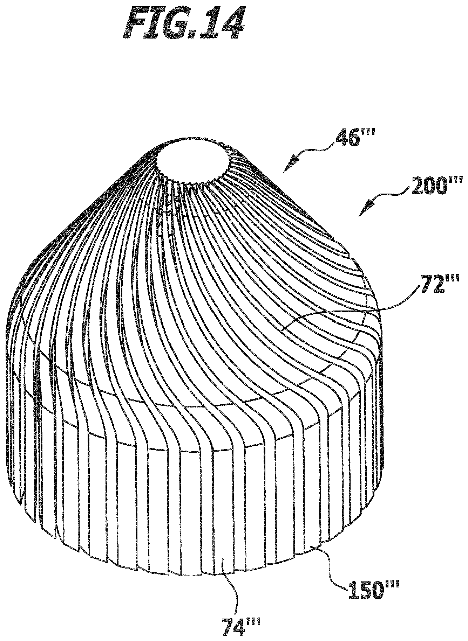

In Figur 14 ist schematisch ein weiteres Ausführungsbeispiel eines zweiten Werkzeugelements dargestellt und insgesamt mit dem Bezugszeichen 46"' bezeichnet. Es unterscheidet sich vom zweiten Werkzeugelement 46 im Wesentlichen dadurch, dass die die Werkzeugelementglieder 74"' trennenden Schlitze 72"' nicht in radialer Richtung von der Längsachse 14 weg orientiert sind, sondern sich gekrümmt von der Längsachse 14 weg erstrecken, so dass die in Figur 14 erkennbare spiralförmige Struktur der Werkzeugelementglieder 74"' gebildet wird. Das zweite Werkzeugelement 46"' kann, insbesondere in ähnlicher Weise wie das zweite Werkzeugelement 46, mit einer Elektrode 58 ausgestattet sein. Es ermöglicht dann, wie die zweiten Werkzeugelemente 46, 46' und 46", ebenfalls, dass eine vom zweiten Werkzeugelement 46"' in der Entfernungsstellung definierte erste Werkzeugumfangslinie kürzer ist als eine vom zweiten Werkzeugelement in der Betriebsstellung definierte zweite Werkzeugumfangslinie. Ferner ermöglicht es das zweite Werkzeugelement 46"' ebenfalls, dass es von der Betriebsstellung, in welcher es in die Annäherungsstellung bringbar ist, in eine Entfernungsstellung bringbar ist, in welcher Entfernungsstellung eine von der Elektrode 58 definierte Rückzugsumfangslinie kürzer ist als eine von der Elektrode 58 in der Annäherungsstellung definierte Annäherungsumfangslinie. Insgesamt wird so ein Faltmechanismus 200"' ausgebildet zum quasi Öffnen und Schließen des zweiten Werkzeugelements 46"' ähnlich einem Regenschirm.

Nicht näher beschrieben und dargestellt zu werden braucht, wie die Elektroden 28 und 58 jeweils elektrisch leitend mit der Anschlussleitung 20 verbunden sind. Vorzugsweise sind sie gegeneinander elektrisch isoliert. Es ist in einfacher Weise möglich, eine elektrische Zuleitung zur Elektrode 58 insbesondere über die Kraftübertragungsglieder 38 und 40 zu realisieren und an den zweiten Werkzeugelementen 46, 46', 46" und 46"' sowie an den Rückhalteeinrichtungen 82, 82' und 82" entsprechend leitende Verbindungen vorzusehen, um die Elektroden 28 und 58 beziehungsweise 58' mit einem HF-Strom beaufschlagen zu können.

## Patentansprüche

1. Chirurgisches Instrument zum Verbinden von Körpergewebe (126, 128) in einem Hohlorgan mit
einem Instrumentenschaft (12) und einem ersten und einem zweiten relativ zueinander axial bewegbaren Werkzeugelement (46, 48), welche jeweils mit zumindest einer, eine Ringform bildenden Elektrode (28, 58) bestückt sind,
wobei das erste Werkzeugelement (48) am distalen Endabschnitt (22) des Instrumentenschafts (12) angeordnet oder ausgebildet ist und das zweite Werkzeugelement (46) distal zum ersten Werkzeugelement (48) positioniert ist und das erste und das zweite Werkzeugelement miteinander über ein erstes stabförmiges Kraftübertragungsglied (38), welches von einem zweiten, hülsenförmigen Kraftübertragungsglied (40) umgeben ist, verbunden sind, wobei die Kraftübertragungsglieder (38, 40) relativ zueinander parallel zu einer Längsachse (14) bewegbar sind,
wobei das zweite Werkzeugelement (46) schirmartig ausgestaltet und derart eingeschnitten ist, dass es eine Anzahl von in Umfangsrichtung beabstandeten, sowie radial auslenkbaren Werkzeugelementgliedern (74) in der Form von in radialer Richtung abstehenden Armen hat, an deren freien Endabschnitten die zumindest eine Elektrode (58) des zweiten Werkzeugelements gehalten ist, und die aus einer schirmartig radial ausgelenkten Betriebsstellung radial in eine Entfernungsstellung eingelenkt werden können,
wobei die zumindest eine Elektrode des zweiten Werkzeugelements (46) als Elektrodenring (60) ausgebildet ist, welcher in radialer Richtung geschlitzt ist und daher einen variablen Umfang aufweist, und
wobei der Elektrodenring hohl ist und einen Elektrodenringkanal (62) definiert, in welchem ein
Elektrodenausgleichselement (70) bewegbar gehalten ist, um voneinander durch einen Schlitz getrennte freie Enden des in radialer Richtung geschlitzten Elektrodenrings miteinander zu verbinden, wodurch es ermöglicht wird, unabhängig von einem Abstand zwischen den freien Enden des Elektrodenrings (60), die voneinander durch den Schlitz getrennt sind, einen durchgehenden Elektrodenring auszubilden.

2. Chirurgisches Instrument nach Anspruch 1, **dadurch gekennzeichnet, dass** eine vom zweiten Werkzeugelement (46) in der Entfernungsstellung definierte erste Werkzeugumfangslinie (156) kürzer ist als eine vom zweiten Werkzeugelement (46) in der Betriebsstellung definierte zweite Werkzeugumfangslinie (158).

3. Chirurgisches Instrument nach Anspruch 1 oder 2, **gekennzeichnet durch** eine Rückhalteeinrichtung (82) zum Sichern des zweiten Werkzeugelements (46) in der Betriebsstellung, wobei die Rückhalteeinrichtung (82) und das zweite Werkzeugelement (46) relativ zueinander bewegbar angeordnet sind.

4. Chirurgisches Instrument nach einem der voranstehenden Ansprüche, **dadurch gekennzeichnet, dass** die Werkzeugelementglieder (74) einen Faltmechanismus (200) zum Überführen des zweiten Werkzeugelements (46) von der Betriebsstellung in die eingelenkte Stellung und/oder umgekehrt bilden.

5. Chirurgisches Instrument nach Anspruch 4, **dadurch gekennzeichnet, dass** der Faltmechanismus (200) ferner ein erstes Kraftübertragungselement (40) zum Übertragen einer Betätigungskraft auf die Rückhalteeinrichtung (82) zum Überführen derselben von der Betriebsstellung in die eingelenkte Stellung und/oder umgekehrt hat.

6. Chirurgisches Instrument nach Anspruch 5, **dadurch gekennzeichnet, dass** die Rückhalteeinrichtung (82) und ein zweites Kraftübertragungselement (38) zum Übertragen einer Betätigungskraft auf das zweite Werkzeugelement (46) zum Überführen desselben von der Entfernungsstellung bei ausgelenkten Werkzeugelementgliedern (74) in eine Annäherungsstellung und/oder umgekehrt relativ bewegbar zueinander angeordnet sind.

7. Chirurgisches Instrument nach Anspruch 5 oder 6, **dadurch gekennzeichnet, dass** die Rückhalteeinrichtung (82) und/oder das erste und/oder das zweite Kraftübertragungselement (38; 40) relativ zum Schaft (12) bewegbar angeordnet sind.

8. Chirurgisches Instrument nach einem der voranstehenden Ansprüche, **dadurch gekennzeichnet, dass** das zweite Werkzeugelement (46) mindestens zwei Werkzeugelementglieder (74) umfasst, welche mit der zweiten Elektrode (58) gekoppelt sind oder einen Teil derselben tragen.

9. Instrument nach einem der vorhergehenden Ansprüche, **dadurch gekennzeichnet, dass** der Elektrodenring mehrere, durch Schlitze voneinander getrennte Elektrodenringabschnitte (164) aufweist, welche jeweils an einem freien Ende (150') eines Werkzeugelementglieds (46¹) angeordnet oder ausgebildet sind,

10. Chirurgisches Instrument nach Anspruch 3, **dadurch gekennzeichnet, dass** das zweite Werkzeugelement (46) eine Vorspanneinrichtung (80) umfasst zum vorspannenden Halten des zweiten Werkzeugelements (46) gegen die Rückhalteeinrichtung (82) in der Betriebsstellung.

11. Chirurgisches Instrument nach Anspruch 10, **dadurch gekennzeichnet, dass** die Vorspanneinrichtung (80) mindestens ein Vorspannglied (78) aufweist, welches vorzugsweise in Form eines mindestens teilweise federelastischen Elements ausgebildet ist und dass vorzugsweise mindestens ein Teil der mindestens zwei Werkzeugelementglieder (78) ein Vorspannglied (76) aufweist oder bildet.

12. Chirurgisches Instrument nach einem der Ansprüche 3 bis 11, **dadurch gekennzeichnet, dass** die Rückhalteeinrichtung (82) einen in radialer Richtung von einer Längsachse (14) des zweiten Werkzeugelements (46) weg weisend wirkenden Anschlag (85) umfasst, an welchem die mindestens zwei Werkzeugelementglieder (74) oder die Elektrode (58) des zweiten Werkzeugelements (46) in der Betriebsstellung direkt oder indirekt anliegen.

## Claims

1. Surgical instrument for binding body tissue (126, 128) in a hollow element comprising an instrument shaft (12) and a first and a second tool element (46, 48), which can be moved axially relative to one another, which are each fitted respectively with at least one electrode (28, 58) forming an annular shape,
wherein the first tool element (48) is arranged or formed on the distal end section (22) of the instrument shaft (12) and the second tool element (46) is positioned distally to the first tool element (48) and the first and the second tool element are joined together by a first rod-like force-transmitting member (38) which is surrounded by a second, sleeve-like force-transmitting member (40), wherein the force-transmitting members (38, 40) can be moved relative to one another parallel to a longitudinal axis (14),
wherein the second tool element (46) is designed to be parasol-like and is cut into such that it has a number of tool element members (74) which are spaced apart from one another in circumferential direction and can be turned out radially in the form of arms projecting in radial direction, at the free end sections of which the at least one electrode (58) of the second tool element is held and which can be turned in from a parasol-like radially turned out operating position radially into a removal position,
wherein the at least one electrode of the second tool element (46) is designed as an electrode ring (60) which is slit in radial direction and therefore has a variable circumference and
wherein the electrode ring is hollow and defines an electrode ring channel (62) in which an electrode equalisation element (70) is held movably in order to connect together free ends of the electrode ring which is slit in radial direction and which ends are separated from one another by a slit, whereby it is possible independently of a distance between the free ends of the electrode ring (60), which are separated from one another by the slit, to form a continuous electrode ring.

2. Surgical instrument according to claim 1, **characterised in that** a first tool perimeter (156) defined by the second tool element (46) in the removal position is shorter than a second tool perimeter (158) defined by the second tool element (46) in the operating position.

3. Surgical instrument according to claim 1 or 2, **characterised by** a retaining device (82) for securing the second tool element (46) in the operating position, wherein the retaining device (82) and the second tool element (46) are arranged to be movable relative to one another.

4. Surgical instrument according to any of the preceding claims, **characterised in that** the tool element members (74) form a folding mechanism (200) for transferring the second tool element (46) from the operating position into the turned in position and/or vice versa.

5. Surgical instrument according to claim 4, **characterised in that** the folding mechanism (200) further comprises a first force-transmitting element (40) for transmitting an activating force to the retaining device (82) for transferring the latter from the operating position into the turned in position and/or vice versa.

6. Surgical instrument according to claim 5, **characterised in that** the retaining device (82) and a second force-transmitting element (38) for transmitting an activating force to the second tool element (46) for transferring the latter from the removal position with turned out tool element members (74) into an approaching position and/or vice versa are arranged to be movable relative to one another

7. Surgical instrument according to claim 5 or 6, **characterised in that** the retaining device (82) and/or the first and/or the second force-transmitting element (38; 40) are arranged to be movable relative to the shaft (12).

8. Surgical instrument according to any of the preceding claims, **characterised in that** the second tool element (46) comprises at least two tool element members (74), which are coupled to the second electrode (58) or support a portion thereof.

9. Surgical instrument according to any of the preceding claims, **characterised in that** the electrode ring comprises a plurality of electrode ring sections (164) separated from one another by slits, which are arranged or formed respectively at a free end (150') of a tool element member (46¹).

10. Surgical instrument according to claim 3, **characterised in that** the second tool element (46) comprises a pretensioning device (80) for pretensioning the second tool element (46) against the retaining device (82) in the operating position.

11. Surgical instrument according to claim 10, **characterised in that** the pretensioning device (80) comprises at least one pretensioning member (78), which is preferably designed in the form of an at least partly spring-resilient element and **in that** preferably at least a portion of the at least two tool element members (78) comprises or forms a pretensioning member (76).

12. Surgical instrument according to any of claims 3 to 11, **characterised in that** the retaining device (82) comprises a stop (85) acting in radial direction pointing away from a longitudinal axis (14) of the second tool element (46), on which stop the at least two tool element members (74) or the electrode (58) of the second tool element (46) bear directly or indirectly in the operating position.

## Revendications

1. Instrument chirurgical pour lier du tissu corporel (126, 128) dans un organe creux avec une gaine d'instrument (12) et un premier et un second élément d'outil (46, 48) déplaçables axialement l'un par rapport à l'autre et qui sont dotés d'au moins une électrode (28, 58) de forme annulaire,
dans lequel le premier élément d'outil (48) est agencé ou conformé dans la section d'extrémité distale (22) de la gaine d'instrument (12) et le second élément d'outil (46) est positionné de manière distale au premier élément d'outil (48) et le premier et le second élément d'outil sont liés l'un à l'autre via un premier chaînon de transmission en forme de tige (38), qui est entouré par un second chaînon de transmission en forme de douille (40), dans lequel les chaînons de transmission (38, 40) peuvent être déplacés l'un par rapport à l'autre parallèlement à un axe longitudinal (14),
dans lequel le second élément d'outil (46) est conformé en écran et découpé de sorte qu'il ait une certain nombre de chaînons d'éléments d'outil (74) espacés dans la direction périphérique et déviables radialement sous la forme de bras éloignés dans la direction radiale, aux sections d'extrémité libres desquelles la au moins une électrode (58) du second élément d'outil est supportée et la position de fonctionnement radialement déviée en forme d'écran peut être radialement guidée en position d'éloignement,
dans lequel la au moins une électrode du second élément d'outil (46) se présente sous la forme d'un anneau d'électrode (60) qui est fendu dans la direction radiale et présente donc une périphérie variable et
dans lequel l'anneau d'électrode est creux et définit un canal d'anneau d'électrode (62) dans lequel un élément de compensation d'électrode (70) est supporté mobile pour lier l'une à l'autre les extrémités libres - séparées l'une de l'autre par une fente - de l'anneau d'électrode fendu dans la direction radiale , si bien que cela permet, indépendamment d'une distance entre les extrémités libres de l'anneau d'électrode (60), qui sont séparées l'une de l'autre par la fente, de former un anneau d'électrode continu.

2. Instrument chirurgical selon la revendication 1, **caractérisé en ce qu'**une première ligne périphérique d'outil (156) définie par le second élément d'outil (46) en position d'éloignement est plus courte qu'une seconde ligne périphérique d'outil (158) définie par le second élément d'outil (46) en position de fonctionnement.

3. Instrument chirurgical selon la revendication 1 ou 2, **caractérisé par** un dispositif de retenue (82) pour fixer le second élément d'outil (46) en position de fonctionnement, dans lequel le dispositif de retenue (82) et le second élément d'outil (46) sont agencés de manière à pouvoir se déplacer l'un par rapport à l'autre.

4. Instrument chirurgical selon l'une quelconque des revendications précédentes, **caractérisé en ce que** les chaînons d'éléments d'outil (74) forment un mécanisme repliable (200) pour transférer le second élément d'outil (46) de la position de fonctionnement en position guidée et/ou vice versa.

5. Instrument chirurgical selon la revendication 4, **caractérisé en ce que** le mécanisme repliable (200) a en outre un premier élément de transmission (40) pour transmettre une force de commande au dispositif de retenue (82) afin de transférer celui-ci de la position de fonctionnement en position guidée et/ou vice versa.

6. Instrument chirurgical selon la revendication 5, **caractérisé en ce que** le dispositif de retenue (82) et un second élément de transmission (38) sont agencés de manière à pouvoir se déplacer l'un par rapport à l'autre pour transférer une force de commande au second élément d'outil (46) afin de transférer celui-ci de la position d'éloignement alors que les chaînons d'éléments d'outil (74) sont déviés en position de rapprochement et/ou vice versa.

7. Instrument chirurgical selon la revendication 5 ou 6, **caractérisé en ce que** le dispositif de retenue (82) et/ou le premier et/ou le second élément de transmission (38 ; 40) sont agencés mobiles par rapport à la gaine (12).

8. Instrument chirurgical selon l'une quelconque des revendications précédentes, **caractérisé en ce que** le second élément d'outil (46) comprend au moins deux chaînons d'élément d'outil (74) qui sont couplés à la seconde électrode (58) ou supportent une partie de celle-ci.

9. Instrument chirurgical selon l'une quelconque des revendications précédentes, **caractérisé en ce que** l'anneau d'électrode présente plusieurs sections d'anneau d'électrode (164) séparées l'une de l'autre par des fentes et qui sont agencées ou conformées respectivement sur une extrémité libre (150') d'un chaînon d'élément d'outil (46¹).

10. Instrument chirurgical selon la revendication 3, **caractérisé en ce que** le second élément d'outil (46) comprend un dispositif de précontrainte (80) pour maintenir sous précontrainte le second élément d'outil (46) contre le dispositif de retenue (82) en position de fonctionnement.

11. Instrument chirurgical selon la revendication 10, **caractérisé en ce que** le dispositif de précontrainte (80) présente au moins un chaînon d'élément de précontrainte (78) qui se présente de préférence sous la forme d'un élément au moins en partie élastique et de préférence au moins une partie des au moins deux chaînons d'éléments d'outil (78) présente ou forme un élément de précontrainte (76).

12. Instrument chirurgical selon l'une quelconque des revendications 3 à 11, **caractérisé en ce que** le dispositif de retenue (82) comprend une butée (85) opérant nommément dans la direction radiale d'un axe longitudinal (14) du second élément d'outil (46), sur laquelle butée les au moins deux chaînons d'élément d'outil (74) ou les électrodes (58) du second élément d'outil (46) s'appliquent directement ou indirectement en position de fonctionnement.
